(19) Europäisches Patentamt
European Patent Office
Office européen des brevets

(11) EP 1 834 642 A2

(12) **EUROPEAN PATENT APPLICATION**

(43) Date of publication:
**19.09.2007 Bulletin 2007/38**

(21) Application number: **07004798.0**

(22) Date of filing: **08.03.2007**

(51) Int Cl.:
*A61K 31/426* (2006.01) *A61K 31/427* (2006.01)
*C07D 417/06* (2006.01) *A61P 31/00* (2006.01)
*A61P 33/06* (2006.01)

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK YU**

(30) Priority: **08.03.2006 IN CH04082006**

(71) Applicant: **National Institute of Immunology**
**New Delhi 110067 (IN)**

(72) Inventors:
- **Surolia, Avadhesha**
  **New Delhi 110067 (IN)**
- **Surolia, Namita**
  **Jakkur, Bangalore**
  **Karnataka, 560 064 (IN)**
- **Kumar, Gyanendra**
  **Bangalore, Karnataka, 560012 (IN)**
- **Chhibber, Manmohan**
  **Bangalore, Karnataka, 560012 (IN)**
- **Parasuraman, Prasanna**
  **Bangalore, Karnataka, 560012 (IN)**
- **Kumar, Sanjay**
  **Bangalore, Karnataka, 560012 (IN)**
- **Sharma, Shailendra K.**
  **Bangalore, Karnataka, 560012 (IN)**
- **Sharma, Shilpi**
  **Bangalore, Karnataka, 560012 (IN)**

(74) Representative: **Ebner von Eschenbach, Jennifer et al**
**LADAS & PARRY LLP**
**Dachauerstrasse 37**
**80335 München (DE)**

(54) **2-thioxothiazolidin-4-one compounds and compositions as antimicrobial and antimalarial agents targeting enoyl-ACP reductase of type II fatty acid synthesis pathway and other cell growth pathways**

(57) The current invention presents enoyl-ACP reductase, an enzyme of the type II fatty acid synthesis pathway as a target for treating human malarias and other infectious diseases. We also present in the current invention, 2-thioxothiazolidin-4-ones as antimicrobial and antimalarial agents. We provide 2-thioxothiazolidin-4-ones as antimicrobial and antimalarial agents either alone or in combination with other known antimicrobial and antimalarial agents with or without added adjuvants or diluents or carriers.

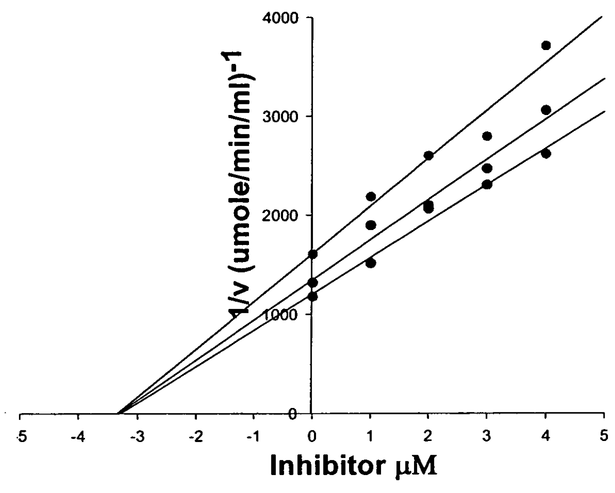


**Fig.1A(i)**



Printed by Jouve, 75001 PARIS (FR)

(Cont. next page)

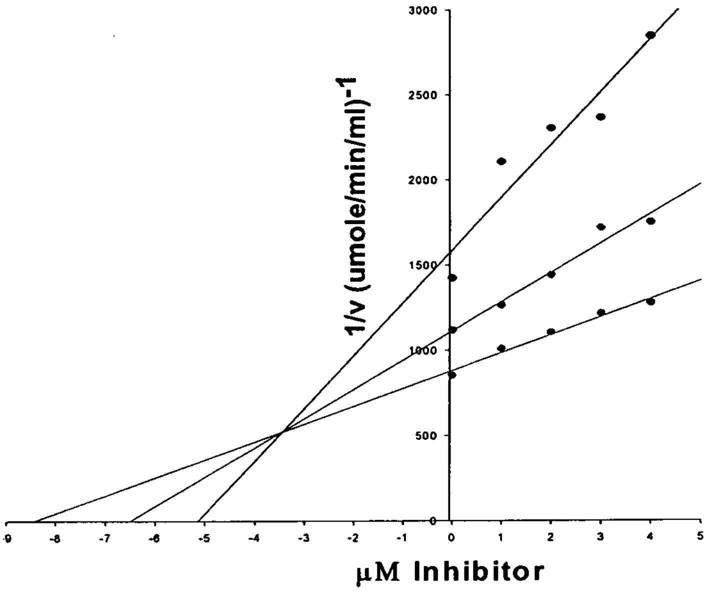
3000
2500
2000
1500
1000
500
0
1/v (umole/min/ml)-1
-9
-8
-7
-6
-5
-4
-3
-2
-1
0
1
2
3
4
5
μM Inhibitor

Fig.1A(ii)

## Description

### Field of Invention

The present invention relates to the 2-thioxothiazolidin-4-one compounds exemplified by NAV-048, NAV-029, NAV-082, NAV-083, NAV-038, NAV-101, NAV-102, NAV-103, NAV105 and NAV-117 as inhibitors of FabI (enoyl ACP reductase) and as novel antimalarial and antibacterial agents either alone or in combination with other known antimalarials or antibacterials and may be formulated with pharmaceutically acceptable adjuvants, excipients, diluents or carriers. The invention further relates to the identification of a drug that exhibits anti-malarial, anti-bacterial or biocidal activity by inhibiting enoyl ACP reductase enzyme, a component of fatty acid synthesis pathway essential for cell growth. Hence the invention also relates to 2-thioxothiazolidin-4-one compounds (NAV-048, NAV-029, NAV-082, NAV-083, NAV-038, NAV-101, NAV-102, NAV-103, NAV105 and NAV-117) as inhibitors of the growth of micro-organisms and the malaria parasite.

### Background of the Invention

Malaria continues to reign in the tropics today, being endemic to around 100 countries in the world (1). Emerging resistance to chloroquine and other currently prescribed drugs limits treatment of malaria today, in particular, cerebral malaria, caused by Plasmodium falciparum (2, 3). The situation therefore warrants the development of new antimalarials.

Our recent demonstration of the type II fatty acid synthesis (FAS) pathway in the malarial parasite, and its inhibition by triclosan, an inhibitor of the rate limiting enzyme of type II FAS, enoyl-ACP reductase, proved the pivotal role played by the fatty acid biosynthesis pathway in the growth and survival of the malarial parasite (4, 5). The essential role of fatty acids and lipids to cell growth and function, and the different type of fatty acid biosynthesis pathway, the type I FAS, occurring in the human host which is distinct from type II FAS of the malarial parasite makes this pathway an attractive drug target for treating malaria and other infections caused by infectious agents that utilize type II FAS for the synthesis of fatty acids and other metabolites for their growth (6, 7).

The type II fatty acid biosynthesis pathway, found in most bacteria and plants, is typified by the existence of distinct enzymes encoded by unique genes for catalyzing each of the four individual chemical reactions required to complete successive cycles of fatty acid elongation (4, 8, 9). This is in contrast to the type I FAS characterized by a multifunctional enzyme catalyzing all the steps of the pathway (10). The initial condensation reaction, catalyzed by β-ketoacyl-ACP synthase III (FabH), condenses acetyl-CoA with malonyl-ACP to form acetoacetyl-ACP. This is followed by reduction and dehydration reactions catalyzed by β-ketoacyl-ACP reductase (FabG) and β-hydroxyacyl-ACP dehydrase (FabA or FabZ), respectively (8, 11 12). There are several enzymes known which are involved in the condensation and dehydration reactions, however, the final step of elongation is catalyzed usually by a single NADH-specific enoyl-ACP reductase (FabI). The resulting acyl-ACP can either be elongated further or be transferred to glycerol phosphate by the acyltransferase system.

FabI is the last enzyme in the cycle, which pulls each cycle of elongation to completion (11, 13). FabI thus presents itselfas a suitable drug target for the design of antimalarials and as an anti-bacterial target in general. Compounds NAV-048, NAV-029, NAV-082, NAV-083, NAV-038, NAV-101, NAV-102, NAV-103, NAV105 and NAV-117 inhibit FabI mediated processing in E. coli bacteria such as the fatty acid synthesis. Hence the present invention represents a major development over the prior art. The fact that growth of bacteria containing PfFabI over-expressing pBAD-PfFabI construct is also inhibited remarkably by these inhibitors indicate that additionally these compounds are able to inhibit the growth of micro-organisms targeting other pathways essential for their growth.

The primary aspect is to present inhibitors of FabI as antimalarial and an antibacterial agent.

Another aspect is to provide antimalarial or antibacterial compositions comprising inhibitors of FabI as exemplified by compounds NAV-048, NAV-029 NAV-082, NAV-083, NAV-038, NAV-101, NAV-102, NAV-103, NAV105 or NAV-117 and NAV-048, NAV-029, NAV-082, NAV-083, NAV-038, NAV-101, NAV-102, NAV-103, NAV105 and NAV-117 themselves, their analogs or their pharmaceutically acceptable derivatives or their pharmaceutically acceptable salts either alone or in combination with other known antimalarials or antibacterials along with pharmaceutically acceptable adjuvants, excipients, diluents or carriers.

### Summary of the Invention

The present method provides a novel approach of treating malaria using compounds NAV-048, NAV-029, NAV-082, NAV-083, NAV-038, NAV-101, NAV-102, NAV-103, NAV105 and NAV-117. None of these compounds which have been shown to possess any inhibitory activity for any known enzyme or any biologic activity prior to this invention. The current method also provides evidence that the compounds NAV-048, NAV-029, NAV-082, NAV-083, NAV-038, NAV-101, NAV-102, NAV-103, NAV105 and NAV-117 abrogate the growth of the malarial parasite in vitro. According to

another aspect of the current invention these classes of compounds can be used as prospective therapy for treating malaria. In yet another aspect of the current invention, evidence for an anti-malarial composition comprising an inhibitor of FabI is provided. In an embodiment of the current invention, evidence for an antimalarial composition comprising compounds NAV-048, NAV-029, NAV-082, NAV-083, NAV-038, NAV-101, NAV-102, NAV-103, NAV105 or NAV-117 or any other inhibitor of FabI either alone or in combination with one or more known antimalarials along with a pharmaceutically acceptable adjuvant, excipient, a diluent or a carrier. The invention also relates to treating a malarial condition caused by a drug-resistant malarial parasite by compounds NAV-048, NAV-029, NAV-082, NAV-083, NAV-038, NAV-101, NAV-102, NAV-103, NAV105, NAV-117 or some other inhibitor of FabI either alone or in combination as mentioned earlier. In another embodiment of the invention, this antimalarial composition may be used either for treating infections caused by *Plasmodium falciparum* or other species of malarial parasites of human or animal origin. In yet another embodiment of the current invention, the antimalarial composition may be used for treating a disease arising out of a parasitic condition caused by any of the organisms of the class Apicomplexa, or pathogenic conditions caused by organisms having the fatty acid biosynthesis system FASII and or requiring FabI enzyme as an essential or a component of its metabolic machinery necessary for their growth.

It is to be understood that both the foregoing general description and the following detailed description are exemplary and explanatory only and are intended to provide further explanation of the invention as claimed. The accompanying drawings are included to provide a further understanding of the invention and are incorporated in and constitute part of this specification.

Brief Description of the Figures

FIG. 1. shows Dixon plots for the inhibition of PtFabI by NAV-048 (A), NAV-082 (B), NAV-083 (C), and NAV-038 (D). Enzyme activity was determined in the presence of 100 μM NADH and 200μM crotonoyl-CoA.

Fig. 1A(i) shows initial rate of *Plasmodium falciparum* enoyl-ACP reductase reaction in the presence of NAV-48 with respect to crotonoyl-CoA. Ki was determined from the x-intercept of Dixon plot assuming non-competetive inhibition.

Fig. 1A(ii) shows initial rate of *Plasmodium falciparum* enoyl-ACP reductase reaction in the presence of NAV-48 with respect to NADH. Ki was determined from the x-intercept of Dixon plot assuming competitive inhibition.

Fig. IB(i) shows initial rate of *Plasmodium falciparum* enoyl-ACP reductase reaction in the presence of NAV-82 with respect to crotonoyl-CoA. Ki was determined from the x-intercept of Dixon plot assuming competitive inhibition.

Fig. 1B(ii) shows initial rate of *P. falciparum* enoyl-ACP reductase reaction in the presence of NAV-82 with respect to NADH. Ki was determined from the x-intercept of Dixon plot assuming non-competitive inhibition.

Fig.1 C(i) shows initial rate of *Plasmodium falciparum* enoyl-ACP reductase reaction in the presence of NAV-83 with respect to crotonoyl-CoA. Ki was determined from the x-intercept of Dixon plot assuming competitive inhibition.

Fig. 1 C(ii) shows initial rate of *Plasmodium falciparum* enoyl-ACP reductase reaction in the presence of NAV-83 with respect to NADH. Ki was determined from the x-intercept of Dixon plot assuming non-competitive inhibition.

Fig. 1D(i) shows initial rate of *Plasmodium falciparum* enoyl-ACP reductase reaction in the presence of NAV-38 with respect to crotonoyl-CoA. Ki was determined from the x-intercept of Dixon plot assuming competitive inhibition.

Fig. 1D(ii) shows initial rate of *Plasmodium falciparum* enoyl-ACP reductase reaction in the presence of NAV-38 with respect to NADH. Ki was determined from the x-intercept of Dixon plot assuming non-competitive inhibition.

FIG. 2 shows inhibitors Fig. 2(A) NAV-048, Fig. 2(B) NAV-029, Fig. 2(C) NAV-082, Fig. 2(D) NAV-083, and Fig. 2 (E) NAV-038, all docked with PfFabI. Inhibitors are shown in ball and sticks, cofactor in sticks and the enzyme in solid ribbon.

Figure 3 shows inhibition of PfENR by compound NAV-117. PfENR activity was determined in the presence of various concentrations of the inhibitor (25 nM to 750 nM). The percent inhibition was calculated from the residual PfENR activity and was plotted against log compound NAV-117 used. The sigmoidal curve indicates the best fit for

the data and $IC_{50}$ value was calculated from the graph.

Figure 4 shows inhibition kinetics of compound NAV-117 with respect to crotonoyl CoA determined using Dixon plot. PfENR was assayed at two fixed concentrations - 100 μM [•] and 200 μM [<] of crotonoyl CoA in presence of 250 nM of compound NAV-117 and 100 μM of NADH. Ki was calculated from the X-intercept. Plot indicates competitive kinetics with respect to crotonoyl CoA.

Figure 5 shows inhibition kinetics of compound NAV-117 with respect to NADH determined using Dixon plot. 200 nM PfENR was assayed in presence of 200 μM crotnoyl CoA, 250 nM compound NAV-117 and 50 μM [•] and 100 μM [<] of NADH. Ki of inhibitor was calculated from the X-intercept. Plot indicates noncompetitive kinetics with respect to NADH.

Figure 6 shows inhibition of growth of the parasite in red blood cell cultures. Compound NAV 117 was assayed for in vitro growth inhibition of *Plasmodium falciparum* by incubating the cultures with different concentrations of the inhibitors in $Me_2SO$ (final concentration, 0.05%). The cultures were checked for growth inhibition by microscopic examination by assessing the parasitemia at every 48 h (•) and 96 h (o). The average of three data sets has been plotted, and the error bars are shown.

The following examples are given by way of illustration of the present invention and should not be construed to limit the scope of present invention.

EXAMPLE 1

Cloning, expression and purification of PfFabI in *E.coli.* The 1296-bp DNA sequence coding for P. falciparum was cloned in pGME-T vector (4). The clone was confirmed by digestion and dideoxy sequencing. For expression purpose, the clone was transformed into *E.coli* BL21 (DE3) cells and cultured overnight. The cells were harvested, lysed and loaded onto a His-bind resin (Novagen). The protein was eluted using step gradient of 0.3-0.5 M imidazole and eluted protein was tested for purity by SDS-PAGE.

EXAMPLE 2

Spectrophotometric assay of enzyme activity The activity of the Enoyl-ACP reductase was assayed by following rate of the disappearance of NADH using crotonyl-CoA as a substrate. The assay mixture (100μl) comprised of 100μM crotonyl-CoA, 100μM NADH and 1% ethanol in 100μM sodium phosphate buffer, pH 7.5. The kinetic parameters were obtained form the double reciprocal plots.

EXAMPLE 3

Enzyme inhibition studies The inhibition studies were carried out by preincubating enoyl ACP reductase with the respective compound and various concentrations of the coenzymes at 4°C for 5 hours. This was warmed to 25°C and assay was started by the addition of the crotonyl -CoA. The inhibition constant Ki was determined by using the following equation:

$$v = v_0 / ( 1+[I]/K_i )$$

where $v_0$ is the unihibited rate and v is the rate in the presence of inhibiting compound.
See Figures 1A(i), 1 A(ii), 1B(i), 1 B(ii), 1 C(i), 1C(ii), 1 D(i) and 1 D(ii).

In Figure 1A(i), tthe dots represent the various concentrations of inhibitor (NAV-48)at a fixed concentration of 50 microM crotonoyl CoA (upper line, ), 100 microM crotonoyl CoA (middle line, ) and 200 microM crotonoyl CoA (lower line).

In Figure 1(A), (ii) the dots represent the various concentrations of inhibitor (NAV-48) at a fixed concentration of 50 microM NADH (upper line, 40), 100 microM NADH (middle line, ) and 200 microM NADH (lower line, ).

In Figure 1B (i), the dots represent various concentrations of inhibitor (NAV-82) at a fixed concentration of 50 (upper line), 100 (middle line), and 200 (lower line) microM of crotonoyl CoA.

In Figure 1 B(ii), the dots represent various concentrations of inhibitor (NAV-82) at a fixed concentration of 50 (upper line), 100 (middle line), and 200 (lower line) microM of NADH.

In Figure 1 C(i), the dots represent various concentrations of inhibitor (NAV-83) at a fixed concentration of 50 (upper line), 100 (middle line), and 200 (lower line) microM of crotonoyl CoA.

In Figure 1C (ii), the dots represent various concentrations of inhibitor (NAV-83) at a fixed concentration of 50 (upper line), 100 (middle line), and 200 (, lower line) microM of NADH.

In Figure 1D (i), the dots represent various concentrations of inhibitor (NAV-38) at a fixed concentration of 50 (, upper line), 100 (, middle line), and 200 (, lower line) microM of crotonoyl CoA.

In Figure 1D(ii), the dots represent various concentrations of inhibitor (NAV-38) at a fixed concentration of 50 (, upper line), 100 (, middle line), and 200 (, lower line) microM of NADH.

EXAMPLE 4

Docking of Substrates and Inhibitors with FabI: Docking simulations were used to determine the binding sites of these compounds in the FabI enzyme. The procedure is described as follows:

Preparation of the ligand and receptor molecules: The crystal structure of PfFabI submitted to PDB (www.resb.org) by Perozzo et al (14) was used for docking studies. Triclosan was removed from the active site of the pdb file 1NHG.pdb and the binary complex (PfFabI with cofactor) was converted into mol2 format with MMFF94 charges loaded using the MOE (Molecular Operating Environment) suite of programs (15). The script molto2pdbqs (provided with AutoDock program) was used to prepare the receptor file. Ligands were built using MOE and energy minimized with MMFF94 charges. The script AutoTors (provided with AutoDock program) was used to define torsion angles in the ligand prior to docking.

Docking simulations: All docking simulations were done with AutoDock (16,17). Briefly, grid maps for docking simulations were generated with 81 grid points (with 0.375 Å spacing) in x, y and z direction centered in the active site using the AutoGrid program. Lennard-Jones parameters 12-10 and 12-6 (supplied with the program package) were used for modeling H-bonds and van der Waals interactions, respectively. The distance-dependent dielectric permittivity of Mehler and Solmajer (18) was used in the calculations of the electrostatic grid maps. The Genetic algorithm (GA) and Lamarckian genetic algorithm with the pseudo-Solis and Wets modification (LGA/pSW) methods were used with default parameters. Each docking experiment consisted of a series of 100 simulations.

EXAMPLE 5

Inhibitory effect of compounds; The inhibitory effect of the compounds, NAV-048, NAV-029, NAV-082, NAV-083, NAV-038, NAV-101, NAV-102, NAV-103, NAV105 and NAV-117 was determined by growing *E. coli* O55:B5 bacteria in Luria broth at 37°C overnight in the presence of various concentrations of respective compounds in 0.5% DMSO. Only 0.5% DMSO was added to the control tube. The inhibitory effect of compounds NAV-048, NAV-029, NAV-082, NAV-083, NAV-038, NAV-101, NAV-102, NAV-103, NAV105 and NAV-117 in bacteria was assessed by the absorbance of the culture at 600nm.

EXAMPLE 6

Kinetics of inhibition of PfENR. We deduced the kinetics of inhibition for NAV-117 with respect to crotonoyl CoA as well as NADH. The reaction velocity was measured at a fixed concentration of substrate varying the inhibitor concentration. A graph of the reciprocal of velocity against inhibitor concentration was plotted. This was repeated at two different substrate concentrations and a similar line drawn for each concentration of substrate. A vertical line droping from the intersection of the two lines on to the inhibitor-axis gives the $K_i$. All these inhibitors were found to be competitive with respect to crotonoyl CoA and noncompetitive with respect to NADH. The $IC_{50}$ value was calculated by ploting the residual PfENR activity against log of inhibitor concentration and fitting the data points into a sigmoidal curve (Figure 3). The IC50 value of NAV-117 was found to be 242 nM. Figure 4 shows inhibition kinetics with respect to crotonoyl CoA. The lines representing the two substrate-concentrations cross each other above the inhibitor-axis indicating competitive inhibition. Figure 5 shows the inhibition kinetics with respect to NADH and in this case the two lines intersect exactly on the inhibitor axis indicating noncompetitive inhibition.

EXAMPLE 7

*In vitro* whole-cell assay against *Plasmodium falciparum.* Antimalarial activity of NAV-117 was checked towards the Chloroquine-sensitive *Plasmodium falciparum* FCK2 strain in red blood cell cultures. The $IC_{50}$ value for growth inhibition of the parasite was found to be - 0.75 μM by non-linear regression analysis after 96 h (Figure 6) and is quite significant. The MIC (minimum inhibitory concentration) value of the compound was found to be 2.5 μM. This shows that rhodanine class of compounds hold great promise to be development into antimalarial agents.

EXAMPLE 8

NAV-048 series of inhibitors: The details of the NAV-048 series of inhibitors are given as follows:

a. Formula 1

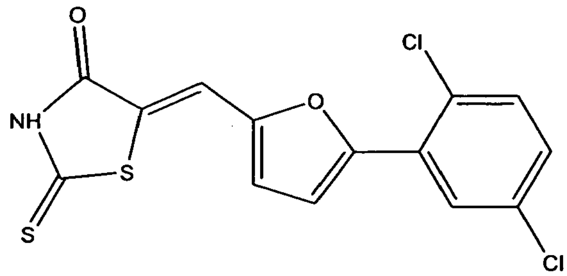

The present invention also describes an antimicrobial and an antimalarial compound, more specifically, NAV-048 (Formula 1) as an inhibitor of FabI enzyme of a bacterial or a malarial or a mycobacterial or a yeast or a fungal source. The chemical formula of the compound is 5-((5-(2,5-dichlorophenyl)furan-2-yl)methylene)-2-thioxothiazolidin-4-one which may also be called as 2,5-dichloro-(5-(4-oxo-2-thioxothiazolidin-5-ylidenemethyl)furan-2-yl)benzene or 2-(4-oxo-2-thioxothiazolidin-5-ylidenemethyl)-5-(2,5-dichlorophenyl) furan.

b. Formula 2

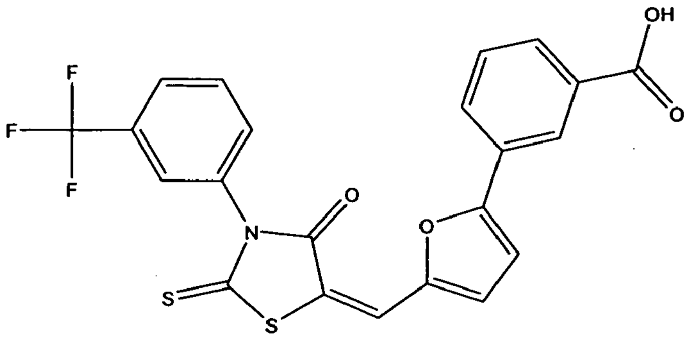

The present invention also describes another antimicrobial and antimalarial compound, more specifically, NAV-029 (Formula 2) as an inhibitor of FabI enzyme of a bacterial or a malarial or a mycobacterial or a yeast or a fungal source. The chemical formula of the compound is 5-(5-(3-carboxyphenyl)furan-2-ylmethylene)-3-(3-trifluoromethylphenyl)-2-thioxothiazolidin-4-one or 5-(5-(3- carboxyphenyl)furan-2-ylmethylene)-N-(3-trifluoromethylphenyl)-2-thioxothiazolidin-4-one. This may also be called as 3-(5-(-(3-(3-(trifluoromethyl)phenyl)-4-oxo-2-thioxothiazolidin-5-ylidene) methyl) furan-2-yl)benzoic acid or 3-(5-(-(N-(3-(trifluoromethyl)phenyl)-4-oxo-2-thioxothiazolidin-5-ylidene) methyl)furan-2-yl)benzoic acid or 2-(3-(3-trifluoromethylphenyl)-4-oxo-2-thioxothiazolidin-5-ylidenemethyl)-5-(3-carboxyphenyl)fizran or 2-(N-(3-trifluoromethylphenyl)-4-oxo-2-thioxothiazolidin-5-ylidenemethyl)-5-(3-carboxyphenyl)furan or 1-(4-oxo-5-((5-(3-carboxyphenyl)furan-2-yl)methylene)-2-thioxothiazolidin-3-yl)-3-trifluoromethylbenzene or 1-(4-oxo-5-((5-(3-carboxyphenyl)furan-2-yl)methylene)-2-thioxothiazolidin-N-yl)-3-trifluoromethylbenzene.

c. Formula 3

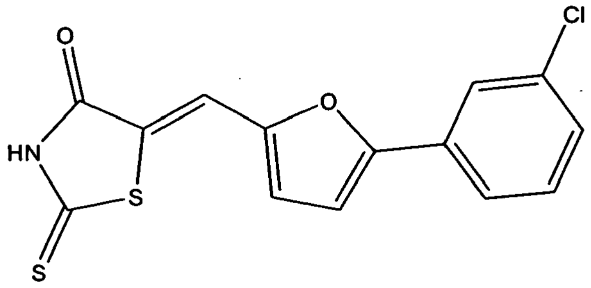

The present invention also describes an antimicrobial and an antimalarial compound, more specifically, NAV-101 (Formula 3) as an inhibitor of FabI enzyme of a bacterial or a malarial or a mycobacterial or a yeast or a fungal source. The chemical formula of the compound is 5-[5-(3-Chloro-phenyl)-furan-2-ylmethylene]-2-thioxo-thiazolidin-4-one.

d. Formula 4

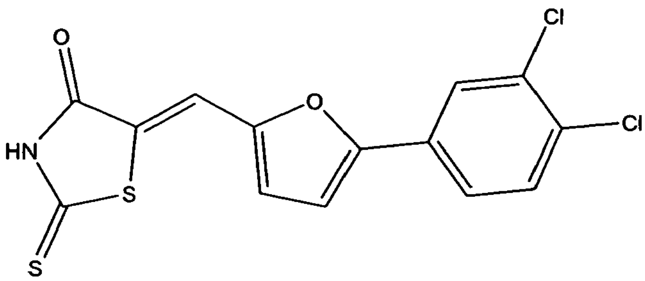

The present invention also describes an antimicrobial and an antimalarial compound, more specifically, NAV-102 (Formula 4) as an inhibitor of FabI enzyme of a bacterial or a malarial or a mycobacterial or a yeast or a fungal source. The chemical formula of the compound is 5-[5-(3,4-Dichloro-phenyl)-furan-2-ylmethylene]-2-thioxo-thiazolidin-4-one.

e. Formula 5

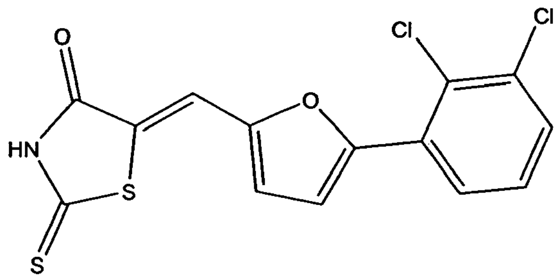

The present invention also describes an antimicrobial and an antimalarial compound, more specifically, NAV-103 (Formula 5) as an inhibitor of FabI enzyme of a bacterial or a malarial or a mycobacterial or a yeast or a fungal source. The chemical formula of the compound is 5-[5-(2,3-Dichloro-phenyl)-furan-2-ylmethylene]-2-thioxo-thiazolidin-4-one.

f. Formula 6

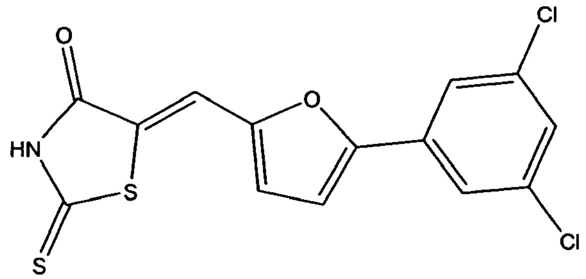

The present invention also describes an antimicrobial and an antimalarial compound, more specifically, NAV-105 (Formula 6) as an inhibitor of FabI enzyme of a bacterial or a malarial or a mycobacterial or a yeast or a fungal source. The chemical formula of the compound is 5-[5-(3,5-Dichloro-phenyl)-furan-2-ylmethylene]-2-thioxo-thiazolidin-4-one.

g. Formula 7

A general formula for the above six series of molecules.

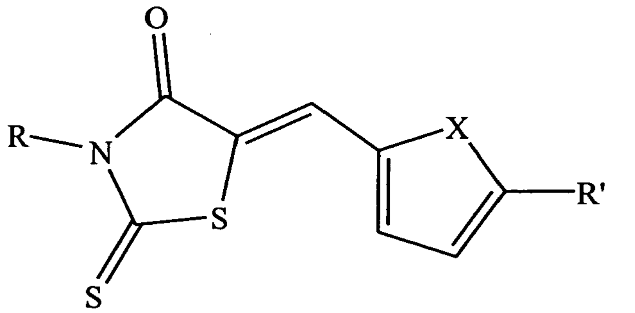

X = O/NH/S

R = H/$CH_3$/-$(CH_2)_n$-COOX (where n is 1/2/3/4/5/6 and X = H/ -$(CH_2)_{n'}$ -$N(CH_3)_2$ where n' = 1/2/3/4/5)/-$(CH_2)_n$-COOH (where n is 1/2/3/4/5/6/)

R = 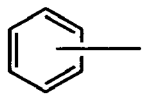 Cl/Br/F/I/$CF_3$/$CCl_3$/$CBr_3$/$CI_3$/$CH_3$/$OCH_3$/Nitro/Amino/ Cyano/Carboxylic/ Aldehydic/Hydroxy/Acyl/Amido/ Alkyl/Alkyloxy

R' = H/-$(CH_2)_n$-COOH (where n is 1/2/3/4/5/6/)

R' = 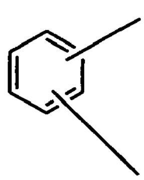

$R_1$ = H/OH/ Cl/ Br/ I/ F/ $CF_3$/$CCl_3$/$CBr_3$/$CI_3$/ $CH_3$/ $OCH_3$/$O(CH_2)_nCH_3$//-$OCOCH_3$/-$COO(CH_2)_nCH_3$/ Nitro/Amino/Cyano/ Carboxylic/Aldehydic/Acyl/ Amido/Alkyl/Alkyloxy ( where n is 1/2/3/4/5)

$R_2$ = H/OH/ Cl/ Br/ I/ F/$CF_3$/$CCl_3$/$CBr_3$/$CI_3$/$CH_3$/$OCH_3$/-$OCOCH_3$/ /-$OCO(CH_2)_nCH_3$/-$COOCH_3$/-$COO(CH_2)_nCH_3$/ Nitro/Amino/Cyano/Carboxylic/Aldehydic/Acyl/Amido/Al kyl/Alkyloxy ( where n is 1/2/3/4/5)

Further the general formula for the above six series of compounds (NAV-048, NAV-029, NAV-101, NAV-102, NAV-103 and NAV-105) is given here (Formula 6). The R group can have H, -$(CH_2)_n$-COOX (where n is 1/2/3/4/5/6 and

X = H/ $-(CH_2)_{n'}-$ $-N(CH_3)_2$ where n' = 1/2/3/4/5)/$-(CH_2)_n-COOH$ (where n is 1/2/3/4/5/6/) or aromatic ring that can have various permutations and combinations with any alkyl or alkyloxy group or halogen atoms e.g. fluoro / chloro / bromo / iodo or nitro or amino or cyano or carboxylic or aldehydic or hydroxy or acyl or amido or $CF_3$ or $CCl_3$ or $CBr_3$ or $CI_3$ group, etc. Similarly R' group can have the one or the other of various permutations and combinations of the R group. Position X can be O, S or NH group.

h. Formula 8

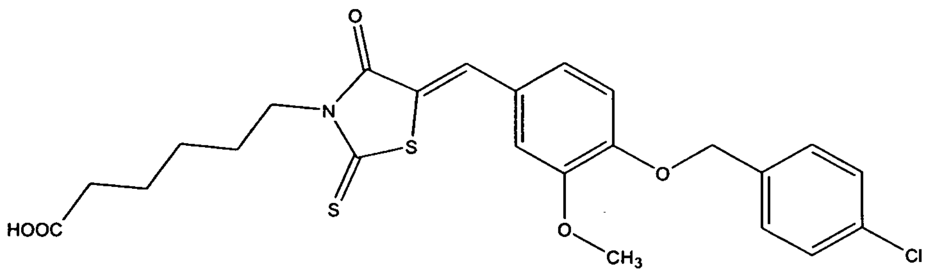

The present invention also describes an antimicrobial and an antimalarial compound NAV-082 (Formula 8) as an inhibitor of FabI enzyme of a bacterial or a malarial or a mycobacterial or a yeast or a fungal source. The chemical formula of the compound is 6-(-5-(4-(4-chlorobenzyloxy)-3-methoxybenzylidene)-4-oxo-2-thioxothiazolidin-3-yl)hexanoic acid or 6-(-5-(4-(4-chlorobenzyloxy)-3-methoxybenzylidene)-4-oxo-2-thioxothiazolidin-N-yl)hexanoic acid or 5-(4-(4-chlorobenzyloxy)-3-methoxybenzylidene)-3-(5-carboxypentyl)-2-thioxothiazolidin-4-one or 5-(4-(4-chlorobenzyloxy)-3-methoxybenzylidene)-N-(5-carboxypentyl)-2-thioxothiazolidin-4-one, which may also be called as 1-(4-oxo-3-(5-carboxypentyl)-2-thioxothiazolidin - 5 - ylidene methyl)- 4 - (4 - chlorobenzyloxy)- 5 - methoxybenzene or 1-(4-oxo-N-(5-carboxypentyl)-2-thioxothiazolidin-5-ylidene methyl)-4-(4-chlorobenzyloxy)-5-methoxybenzene or 1-((2-Methoxy-4-(4-oxo-3-(5-carboxypentyl)-2-thioxothiazolidin-5-ylidene methyl)phenoxy) methyl)-4-chlorobenzene or 1-((2-Methoxy-4-(4-oxo-N-(5-carboxypentyl)-2-thioxothiazolidin-5-ylidenemethyl) phenoxy) methyl)-4-chlorobenzene.

i. Formula 9

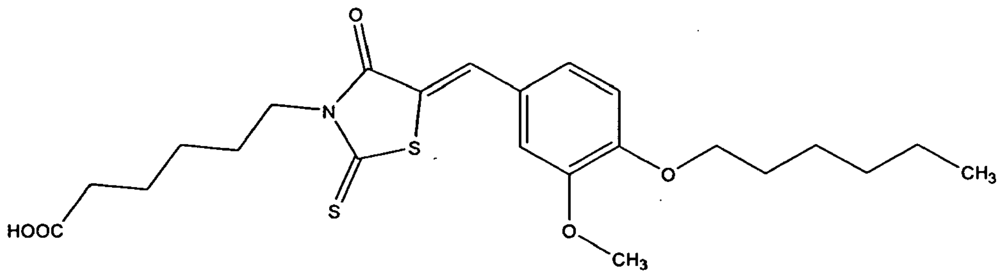

The present invention also describes an antimicrobial and an antimalarial compound NAV-083 (Formula 9) as an inhibitor of FabI enzyme of a bacterial or a malarial or a mycobacterial or a yeast or a fungal source. The chemical formula of the compound is 6-(-5-(4-(hexyloxy)-3-methoxybenzylidene)-4-oxo-2-thioxothiazolidin-3-yl)hexanoic acid or 6-(-5-(4-(hexyloxy)-3-methoxybenzylidene)-4-oxo-2-thioxothiazolidin-N-yl)hexanoic acid. This may also be called as 5-(4-Hexyloxy-3-methoxybenzylidene)-3-(5-carboxypentyl)-2-thioxothiazolidin-4-one or 5-(4-Hexyloxy-3-methoxybenzylidene)-N-(5-carboxypentyl)-2-thioxothiazolidin-4-one or 1-(4-oxo-3-(S-carboxypentyl)-2-thioxothiazolidin-5-ylidene methyl)-4-hexyloxy-3-methoxy benzene or 1-(4-oxo-N-(5-carboxypentyl)-2-thioxothiazolidin-5-ylidene methyl)-4-hexyloxy-3-methoxy benzene.

j. Formula 10

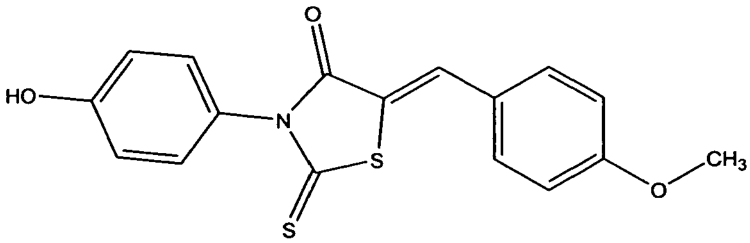

The present invention also describes an antimicrobial and an antimalarial compound NAV-038 (Formula 10) as an inhibitor of FabI enzyme of a bacterial or a malarial or a mycobacterial or a yeast or a fungal source. The chemical formula of the compound is S-(4-methoxybenzylidene)-3-(4-hydroxyphenyl)-2-thioxothiazolidin-4-one or S-(4-methoxybenzylidene)-N-(4-hydroxyphenyl)-2-thioxothiazolidin-4-one. This may also be called as 4-Hydroxy-(5-(4-methoxybenzylidene)-4-oxo-2-thioxothiazolidin-3-yl)benzene or 4-Hydroxy-(5-(4-methoxybenzylidene)-4-oxo-2-thioxothiazolidin-N-yl)benzene or 1-(4-oxo-3-(4-hydroxyphenyl)-2-thioxothiazolidin-5-ylidene methyl)-4-methoxy benzene or 1-(4-oxo-N-(4-hydroxyphenyl)-2-thioxothiazolidin-5-ylidene methyl)-4-methoxy benzene.

k. Formula 11

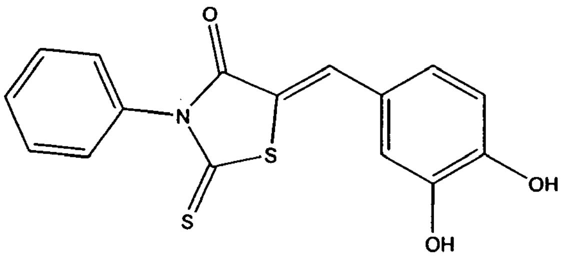

The present invention also describes an antimicrobial and an antimalarial compound NAV-117 (Formula 11) as an inhibitor of FabI enzyme of a bacterial or a malarial or a mycobacterial or a yeast or a fungal source. The chemical formula of the compound is 5-(3,4-Dihydroxy-benzylidene)-3-phenyl-2-thioxo-thiazolidin-4-one.

1. Formula 12

A general formula for the above four series of molecules.

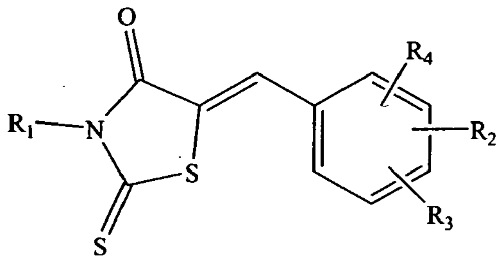

$R_1$ = H/ -$(CH_2)_n$-COOX (where n is 1/2/3/4/5/6 and X = H, -$(CH_2)_{n'}$-$N(CH_3)_2$ where n' = 1/2/3/4/5)

$R_1$ = 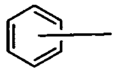 Cl/Br/F/I/$CF_3$/$CCl_3$/$CBr_3$/$CI_3$/$CH_3$/$OCH_3$/$OCH_2CH_3$/$O(CH_2)_{n2}CH_3$/-$N(CH_3)_2$/ -$N(CH_2CH_3)_2$/Nitro/Amino/Cyano/Carboxylic/Aldehydic/Hydroxy/Acyl/Amido/Alkyl/Alkyloxy/-benzyloxy/ benzoyloxyl

$R_2$= H/OH/-Cl/Br/F/I/$CF_3$/$CCl_3$/$CBr_3$/$CI_3$/$CH_3$/$OCH_3$/$OCH_2CH_3$/-$O(CH_2)_nCH_3$/-$N(CH_3)_2$/ -$N(CH_2CH_3)_2$/Nitro/ Amino /fused phenyl ring/fused Dioxolane ring (where n = 0/1/2/3)

$R_3$ = H/OH/$OCH_3$/-$O(CH_2)nCH_3$/-$N(CH_3)_2$/-$N(CH_2CH_3)_2$/-$(CH_2)_n$-COOH/ -O-$(CH_2)_n$-COOH (where n is 1/2/3/4/5/6/)
$R_3$ = Cl/Br/F/I/$CF_3$/$CCl_3$/$CBr_3$/$CI_3$/$CH_3$/$SCH_3$/Nitro/Amino/Cyano/Carboxylic/Aldehydic/H ydroxy/Acyl/Amido/Alkyl/Alkyloxy/-benzyloxy/benzoyloxyl/fused phenyl ring/fused Dioxolane ring

$R_3$ = 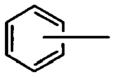 H/Cl/Br/F/I/$CF_3$/$CCl_3$/$CBr_3$/$CI_3$/-$CH_3$/-$N(CH_3)_2$/-$N(CH_2CH_3)_2$/Nitro/Amino/Cyano/Carboxylic/Aldehydic/Hydroxy/Acyl/Amido/Alkyl/Alkyloxy/-benzylo xy/ benzoyloxyl

$R_4$ = H/Cl/Br/F/I/-$OC_6H_5$/-benzyloxy or benzoyloxyl/-$OCH_2C_6H_4Cl$/$OCOC_6H_4CH_3$ /Nitro/Amino/Cyano/Carboxylic/Aldehydic/Hydroxy/Acyl/Amido/Alkyl/Alkyloxy/ fused phenyl ring/fused Dioxolane ring

Further the general formula for the above four series of compounds (NAV-082, NAV-083, NAV-038 and NAV-117) is given here (Formula 12). The $R_1$ group can have H, - $(CH_2)_n$-COOX (where n is 1/2/3/4/5/6 and X = H, -$(CH_2)_{n'}$-$N(CH_3)_2$ where n' = 1/2/3/4/5) or aromatic ring that can have various permutations and combinations with any alkyl or alkyloxy group or halogen atoms e.g. fluoro / chloro / bromo / iodo or nitro or amino or cyano or carboxylic or aldehydic or hydroxy or acyl or amido or $CF_3$ or $CCl_3$ or $CBr_3$ or $CI_3$ or dimethyl amino or diethyl amino group, etc. Similarly $R_3$ group can have H or -$(CH_2)_n$-COOH or -O-$(CH_2)_n$-COOH (where n is 1/2/3/4/5/6) or any alkyl or alkyloxy group or halogen atoms e.g. fluoro / chloro / bromo / iodo or nitro or amino or cyano or carboxylic or aldehydic or hydroxy or acyl or amido or $CF_3$ or $CCl_3$ or $CBr_3$ or $CI_3$ or dimethyl amino or diethyl amino group or an aromatic ring that can have various permutations and combinations with any alkyl or alkyloxy group or halogen atoms e.g. fluoro / chloro / bromo / iodo or nitro or amino or cyano or carboxylic or aldehydic or hydroxy or acyl or amido or $CF_3$ or $CCl_3$ or $CBr_3$ or $CI_3$ or dimethyl amino or diethyl amino group or fused phenyl ring, etc with one or the other of various permutations and combinations of the $R_1$ group. $R_2$ can have H or OH or O-$(CH_2)_n$-$CH_3$ (where n = 0/1/2/3) or dimethyl amino or diethyl amino group or fused phenyl ring or dioxolane ring with aromatic system with various permutations and combinations at $R_1$ and $R_3$. And $R_4$ group can have H or OH or any alkyl or alkyloxy group or halogen atoms e.g. fluoro/chloro/ bromo/ iodo or nitro or amino or cyano/ carboxylic/aldehydic/acyl/ amido $CF_3$ or $CCl_3$ or $CBr_3$ or $CI_3$ or dimethyl amino or diethyl amino group or / fused phenyl ring or dioxolane ring or benzyloxy or benzoyloxy/-$OCOC_6H_4Cl$/-$OCH_2C_6H_4Cl$ etc. with one or the other of various permutations and combinations of the $R_1$ group.

As used herein, alkyl is a straight chain or branched aliphatic residue. The alkyl is $C_1$ - $C_{12}$ alkyl, preferably $C_1$ - $C_8$ alkyl.

Alkoxy is a radical O-alkyl where alkyl is as described above.

Acyl is an organic acid group in which the OH of the carboxyl group is replaced by some other substituent. Examples of acyl groups are acetyl, $CH_3CO$, benzoyl, and $C_6H_5CO$.

An aldehydic group contains an aldehyde group CHO.

The compounds of the present invention in accordance with the present invention are useful in the treatment malaria and diseases and disorders associated with malaria or a Plasmodium parasite and other infectious diseases.

The antimalarial of the compounds of the present invention may be measured by any of the methods available to those skilled in the art, including in vitro and in vivo assays. Examples of suitable assays for activity measurements are provided herein. Properties of the compounds of the present invention may be assessed, for example, by using one or more of the assays set out in the Examples below. Other pharmacological methods may also be used to determine the efficacy of the compounds as antimalarial agents.

The compounds of the present invention may be used in combination with or as a substitution for treatments of the above conditions. For example, the compounds of the present invention may also be used alone or combination with antimalarial agents known in the art. The compounds of the present invention may be used alone or in combination with supplementary active compounds including antibiotics, antiprotozoal agents, antifungal agents, and antiproliferative agents, and analgesics known in the art.

The compounds of the present invention, while effect themselves, may be formulated and administered in the form of their pharmaceutically acceptable salts, such as acid addition salts or base addition salts, for purposes of stability, convenience of crystallization, increased solubility and the like. For the purpose of this invention, a pharmaceutical composition will contain one or more of the active compounds of the invention, their derivatives, salts, pro-drugs and/or hydrates thereof, in a form to be administered alone, but generally in a form to be administered in admixture with a pharmaceutical carrier selected with regard to the intended route of administration and standard pharmaceutical practice. Suitable carriers which can be used are, for example, diluents or excipients such as fillers, extenders, binders, emolllients, wetting agents, disintergrants, surface active agents and lubricants which are usually employed to prepare such drugs depending on the type of dosage form.

The term "pharmaceutically acceptable salts" refers to salt forms that are pharmacologically acceptable and

substantially non-toxic to the subject being treated with the compound of the invention. Pharmaceutically acceptable salts include conventional acid-addition salts or base-addition salts formed from suitable non-toxic organic or inorganic acids or inorganic bases. Exemplary acid-addition salts include those derived from inorganic acids such as hydrochloric acid, hydrobromic acid, hydroiodic acid, sulfuric acid, sulfamic acid, phosphoric acid, and nitric acid, and those derived from organic acids such as p-toluenesulfonic acid, methanesulfonic acid, ethane-disulfonic acid, isethionic acid, oxalic acid, p-bromophenylsulfonic acid, carbonic acid, succinic acid, citric acid, benzoic acid, 2-acetoxybenzoic acid, acetic acid, phenylacetic acid, propionic acid, glycolic acid, stearic acid, lactic acid, malic acid, tartaric acid, ascorbic acid, maleic acid, hydroxymaleic acid, glutamic acid, salicylic acid, sulfanilic acid, and fumaric acid. Exemplary base-addition salts include those derived from ammonium hydroxides (e.g., a quaternary ammonium hydroxide such as tetramethylammonium hydroxide), those derived from inorganic bases such as alkali or alkaline earth-metal (e.g., sodium, potassium, lithium, calcium, or magnesium) hydroxides, and those derived from non-toxic organic bases such as basic amino acids.

The salt may be prepared by methods known in the art.

"A pharmaceutically acceptable prodrug" is a compound that may be converted under physiological conditions or by solvolysis to the specified compound or to a pharmaceutically acceptable salt of such compound. "A pharmaceutically active metabolite" is intended to mean a pharmacologically active product produced through metabolism in the body of a specified compound or salt thereof. Prodrugs and active metabolites of a compound may be identified using routine techniques known in the art See, e.g., Bertolini, G. et al., (1997) J. Med. Chem. 40:2011 2016; Shan, D. et al., J. Pharm. Sci., 86(7):765 767; Bagshawe K., (1995) Drug Dev. Res. 34:220 230; Bodor, N., (1984) Advances in Drug Res. 13:224 331; Bundgaard, H., Design of Prodrugs (Elsevier Press, 1985); and Larsen, I. K., Design and Application of Prodrugs, Drug Design and Development (Krogsgaard-Larsen et al., eds., Harwood Academic Publishers, 1991).

It is recognized by one skilled in the art that a described herein can be used to treat an infectious disease or malaria by treating a patient presently afflicted with the disease or condition or can be used to prophylactically treat a patient. A patient who is treated prophylactically is a patient at risk for being exposed to an infectious disease or an organism that causes malaria. Such a patient could be a health care worker, a patient prior to or following surgery or living in or traveling to a location where exposure to an organism causing malaria could occur.

Pharmaceutical compositions suitable for use in the present invention include compositions wherein the active ingredients are contained in an effective amount to achieve its intended purpose.

In addition to the active ingredients, these pharmaceutical compositions may contain suitable pharmaceutically acceptable carriers, excipients, diluents, solvents, binders, flavorings, colorants etc. The preparations may be formulated in any form including but not limited to tablets, capsules, lozenges, powders, aerosols, inhalants, suppositories, syrups, suspensions, slurries, time release formulations, sustained release formulations, pills, granules, emulsions, patches, injections, solutions, liposomes and nanoparticles. The pharmaceutical formulations of the invention may be manufactured in manners generally known for preparing pharmaceutical compositions, e.g., using conventional techniques such as mixing, dissolving, granulating, dragee-making, levigating, emulsifying, encapsulating, entrapping or lyophilizing. Pharmaceutical formulations may be formulated in a conventional manner using one or more physiologically acceptable carriers, which may be selected from excipients and auxiliaries that facilitate processing of the active compounds into preparations which can be used pharmaceutically.

The compositions of this invention are conventionally prepared as tablets, capsules, powders, aerosols, inhalants, suppositories, syrups, suspensions, slurries, time release formulations, sustained release formulations, pills, granules, emulsions, patches, injections, solutions, liposomes and nanoparticlestablets, capsules, powders, aerosols, inhalants, suppositories, syrups, suspensions, slurries, time release formulations, sustained release formulations, pills, granules, emulsions, patches, injections, solutions, liposomes and nanoparticles or other forms depending on the manner of administration. The nature of the pharmaceutical composition employed will, of course, depend on the desired route of administration. Suitable routes of administration are those known in the art and include, oral, inhalation, rectal, transdermal, vaginal, transmucosal or intestinal administration; parenteral delivery, including intramuscular, subcutaneous, intramedullary injections, as well as intrathecal, direct intraventricular, intravenous, intraperitoneal, intranasal, or intraocular injections. The composition may be administered either alone or as a mixture with other therapeutic agents.

The exact formulation, route of administration and dosage can be chosen by the individual physician in view of the patient's condition. In accordance with good clinical practice, it is preferred to administer the composition at a dose that will produce desired effects without causing undue harmful side effects.

The term "an effective amount" means the amount of the drug or pharmaceutical agent that will elicit the biological or medical response of a tissue system, animal or human that is being sought.

A therapeutically effective amount can be readily determined by the attending diagnostician, as one skilled in the art, by using conventional techniques and by observing results obtained under analogous circumstances. In determining the therapeutically effect amount, the dose, a number of factors are considered by the attending diagnostician, including, but not limited to: the species of patient; its size, age, and general health; the specific disease involved; the degree of or involvement or the severity of the disease; the response of the individual patient; the particular compound administered; the mode of administration; the bioavailability characteristic of the preparation administered; the dose

regimen selected; the use of concomitant medication; and other relevant circumstances.

A compound of the present invention may be administered in a therapeutically effective amount to a mammal such as a human. Therapeutically effective amounts of the compounds of the present invention may be used to treat, modulate, attenuate, reverse, or affect malaria in a mammal. An "effective amount" is intended to mean that amount of an agent that is sufficient to treat, prevent, or inhibit malaria or a disease or disorder associated with malaria. In some preferred embodiments, malaria or the disease or disorder associated with malaria is caused by a Plasmodium parasite, preferably, P. falciparum, P. vivax, P. ovale, or P. malariae.

For example, a therapeutically effective amount of a compound of the invention ranges from about 0.1 to about 1,000 mg/kg body weight, preferably about 0.1 to about 500 mg/kg body weight, and more preferably about 0.1 to about 100 mg/kg body weight. The skilled artisan will appreciate that certain factors may influence the dosage required to effectively treat a subject, including but not limited to the severity of the disease or disorder, previous treatments, the general health and/or age of the subject, and other diseases present.

Moreover, treatment of a subject with a therapeutically effective amount of the compound of the present invention may consist of a single administration, or alternatively comprise a series of applications. For example, a subject may be treated with a compound of the present invention at least once. However, the subject may treated with the compound from about one time per week to about once daily for a given treatment period. The length of the treatment period will depend on a variety of factors such as the severity of inflammation, the concentration and activity of the compounds of the present invention, or a combination thereof. It will also be appreciated that the effective dosage of the compound used for treatment may increase or decrease over the course of a particular treatment. Changes in dosage may result and become apparent by standard diagnostic assays known in the art. In some instances chronic administration may be required. The compounds of the present invention may be administered before, during, after, or a combination thereof exposure to malaria or a Plasmodium parasite.

The specification for the dosage unit forms of the invention are dictated by and directly dependent on the unique characteristics of the active compound and the particular therapeutic effect to be achieved, and the limitations inherent in the art of compounding such an active compound for the treatment of individuals.

Toxicity and therapeutic efficacy of such compounds can be determined by standard pharmaceutical procedures in cell cultures or experimental animals, e.g., for determining the LD.sub.50 (the dose lethal to 50% of the population) and the ED.sub.50 (the dose therapeutically effective in 50% of the population). The dose ratio between toxic and therapeutic effects is the therapeutic index and it can be expressed as the ratio LD.sub.50/ED.sub.50. Compounds which exhibit large therapeutic indices are preferred. While compounds that exhibit toxic side effects may be used, care should be taken to design a delivery system that targets such compounds to the site of affected tissue in order to minimize potential damage to uninfected cells and, thereby, reduce side effects.

The data obtained from the cell culture assays and animal studies can be used in formulating a range of dosage for use in humans. The dosage of such compounds lies preferably within a range of circulating concentrations that include the ED.sub.50 with little or no toxicity. The dosage may vary within this range depending upon the dosage form employed and the route of administration utilized. For any compound used in the method of the invention, the therapeutically effective dose can be estimated initially from cell culture assays. A dose may be formulated in animal models to achieve a circulating plasma concentration range that includes the IC.sub.50 (i.e., the concentration of the test compound which achieves a half-maximal inhibition of symptoms) as determined in cell culture. Such information can be used to more accurately determine useful doses in humans. Levels in plasma may be measured, for example, by high performance liquid chromatography.

Embodiments of the invention described herein include:

1. NAV-048 for treating an infectious disease.

2. Derivatives or analogs of NAV-048 or any compound of the NAV-048 class for treating an infectious disease.

3. NAV-029 for treating an infectious disease.

4. NAV-029 derivatives or analogs or any compound of the NAV-029 class for treating an infectious disease.

5. NAV-101 for treating an infectious disease.

6. NAV-101 derivatives or analogs or any compound of the NAV-101 class for treating an infectious disease.

7. NAV-102 for treating an infectious disease.

8. NAV-102 derivatives or analogs or any compound of the NAV-102 class for treating an infectious disease.

9. NAV-103 for treating an infectious disease.

10. NAV-103 derivatives or analogs or any compound of the NAV-103 class for treating an infectious disease.

11. NAV-105 for treating an infectious disease.

12. NAV-105 derivatives or analogs or any compound of the NAV-105 class for treating an infectious disease.

13. NAV-082 for treating an infectious disease.

14. NAV-082 derivatives or analogs or any compound of the NAV-082 class for treating an infectious disease.

15. NAV-083 for treating an infectious disease.

16. NAV-083 derivatives or analogs or any compound of the NAV-083 class for treating an infectious disease.

17. NAV-038 for treating an infectious disease.

18. NAV-038 derivatives or analogs or any compound of the NAV-038 class for treating an infectious disease.

19. NAV-117 for treating an infectious disease.

20. NAV-117 derivatives or analogs or any compound of the NAV-117 class for treating an infectious disease.

21. Enoyl ACP reductase enzyme in P. falciparum as a drug target for developing antimalarial compounds.

22. Enoyl ACP reductase enzyme in a bacterium as a drug target for developing antibacterial compounds.

23. An antimalarial composition wherein the inhibitor of fatty acid synthesis used is (E and Z)-5-((furan-2-yl)methylene)-2-thioxothiazolidin-4-one of general formula 7, wherein the R group can have H, $-(CH_2)_n$-COOH (where n is 1/2/3/4/5/6) or aromatic ring that can have various permutations and combinations with any alkyl or alkyloxy group or halogen atoms e.g. fluoro / chloro / bromo / iodo or nitro or amino or cyano or carboxylic or aldehydic or hydroxy or acyl or amido or $CF_3$ or $CCl_3$ or $CBr_3$ or $CI_3$ group, etc. Similarly R' group can have the one or the other of various permutations and combinations of the R group. Position X can be O, S or NH group.

24. An antimalarial composition as in # 23, wherein R is H, R' is 2,5-dichlorobenzene $((C_6H_5)$-2,5-$C_2)$ and X is O as exemplified by formula 1 (NAV-048).

25. An antimalarial composition as in # 23, wherein R is 3-trifluoromethyl benzene $((C_6H_5)$-3-$CF_3)$, X is O and R' is 3-carboxy phenyl $((C_6H_5)$-3-COOH) as exemplified by formula 2 (NAV-029)

26. An antimalarial composition as in # 23, wherein R is H, X is O and R' is 3-chlorobenzene as exemplified by formula 3 (NAV-101)

27. An antimalarial composition as in # 23, wherein R is H, X is O and R' is 3,4-dichlorobenzene as exemplified by formula 4 (NAV-102)

28. An antimalarial composition as in # 23, wherein R is H, X is O and R' is 2,3-dichlorobenzene as exemplified by formula 5 (NAV-103).

29. An antimalarial composition as in # 23, wherein R is H, X is O and R' is 3,5-dichlorobenzene as exemplified by formula 6 (NAV-105).

30. An antimicrobial composition wherein the inhibitor of fatty acid synthesis used is (E and Z)-5-benzylidene-2-thioxothiazolidin-4-one of general formula 12 wherein $R_1$ group can have H, $-(CH_2)_n$-COOX (where n is 1/2/3/4/5/6 and X = H, - $(CH_2)_{n'}$- $-N(CH_3)_2$ where n' = 1/2/3/4/5) or aromatic ring that can have various permutations and combinations with any alkyl or alkyloxy group or halogen atoms e.g. fluoro / chloro / bromo / iodo or nitro or amino or cyano or carboxylic or aldehydic or hydroxy or acyl or amido or $CF_3$ or $CCl_3$ or $CBr_3$ or $CI_3$ or dimethyl amino or

diethyl amino group, etc. Similarly $R_3$ group can have H or $-(CH_2)_n-COOH$ or $-O-(CH2)_n-COOH$ (where n is 1/2/3/4/5/6) or any alkyl or alkyloxy group or halogen atoms e.g. fluoro / chloro / bromo / iodo or nitro or amino or cyano or carboxylic or aldehydic or hydroxy or acyl or amido or $CF_3$ or $CCl_3$ or $CBr_3$ or $CI_3$ or dimethyl amino or diethyl amino group or an aromatic ring that can have various permutations and combinations with any alkyl or alkyloxy group or halogen atoms e.g. fluoro / chloro / bromo / iodo or nitro or amino or cyano or carboxylic or aldehydic or hydroxy or acyl or amido or $CF_3$ or $CCl_3$ or $CBr_3$ or $CI_3$ or dimethyl amino or diethyl amino group or fused phenyl ring, etc with one or the other of various permutations and combinations of the $R_1$ group. $R_2$ can have H or OH or $O-(CH_2)_n-CH_3$ (where n = 0/1/2/3) or dimethyl amino or diethyl amino group or fused phenyl ring or dioxolane ring with aromatic system with various permutations and combinations at $R_1$ and $R_3$. And $R_4$ group can have H or OH or any alkyl or alkyloxy group or halogen atoms e.g. fluoro/chloro/ bromo/ iodo or nitro or amino or cyano/ carboxylic/aldehydic/ acyl/ amido $CF_3$ or $CCl_3$ or $CBr_3$ or $CI_3$ or dimethyl amino or diethyl amino group or / fused phenyl ring or dioxolane ring or benzyloxy or benzoyloxy/$-OCOC_6H_4Cl$/$-OCH_2C_6H_4Cl$ etc. with one or the other of various permutations and combinations of the $R_1$ group.

31. An antimalarial composition as in # 30, wherein the $R_1$ group can have 5-carboxy pentyl ($-(CH_2)_5-COOH$), $R_2$ can have $-OCH_3$ and $R_3$ can have -4-chloro benzyloxy ($-OCH_2(C_6H_5)-4-Cl$) group as exemplified by formula 8 (NAV-082).

32. An antimalarial composition as in # 30, wherein the $R_1$ group can have 5-carboxy pentyl ($-(CH_2)_5-COOH$), $R_2$ can have $-OCH_3$ and $R_3$ can have 4-hexyloxy ($-O(CH_2)_5-CH_3$) group as exemplified by formula 9 (NAV-083).

33. An antimalarial composition as in # 30, wherein the $R_1$ group can have 4-hydroxyphenyl ($-(C_6H_5)-4-OH$), $R_2$ can have H and $R_3$ can have $-OCH_3$ group as exemplified by formula 10 (NAV-038).

34. An antimalarial composition as in # 30, wherein the $R_1$ group can have phenyl, $R_2$ can have OH and $R_3$ can have OH group as exemplified by formula 11 (NAV-117).

35. An antimalarial composition according to # 23 and # 30 wherein the *Plasmodium falciparum* is resistant to chloroquine or to other currently prescribed drugs.

36. An antimalarial composition according to # 23 and # 30 wherein the enoyl ACP reductase enzyme belongs to any *Plasmodium* species of human or animal origin.

37. Enoyl ACP reductase enzyme as a drug target in developing drugs against any of the pathogenic members of the class Apicomplexa.

38. Enoyl ACP reductase enzyme as a drug target in developing drugs against pathogenic organisms having the type II FAS with a functional FabI or its analogs.

39. Inhibitors of Enoyl ACP reductase in treating malaria caused by *Plasmodium falciparum.*

40. Inhibitors as in # 24 wherein the organism may be *Plasmodium* of any human or animal origin.

41. Inhibitors as in # 24 wherein the organism may be any organism causing a parasitic infection belonging to the class Apicomplexa.

42. Inhibitors as in # 24 wherein the organism may be any pathogenic organism with a type II FAS and having the enoyl ACP reductase enzyme (FabI or its analogs).

43. An antimalarial composition comprising NAV-048, NAV-029, NAV-082, NAV-083, NAV-038, NAV-101, NAV-102, NAV103, NAV105 or NAV-117 or their pharmaceutically acceptable derivatives either alone or in combination with one or more known antimalarials alone or along with a pharmaceutically acceptable adjuvant or a diluent or a carrier.

44. An antimalarial composition, comprising an inhibitor of enoyl ACP reductase (FabI) or its pharmaceutically acceptable derivatives either alone or in combination with one or more known antimalarials alone or along with a pharmaceutically acceptable adjuvant or a diluent or a carrier.

45. An antimalarial composition as in # 28 or # 29 wherein the malaria may be caused by *Plasmodium falciparum* or by any other species of *Plasmodium* of human or animal origin.

46. Use as in # 28 or # 29 wherein the composition may be used in treating any parasitic condition caused by any organism of the class Apicomplexa.

47. Use as in # 28 or # 29 wherein the composition may be used in treating any pathogenic condition caused by any organism having the type II FAS with a functional enoyl ACP reductase, i.e. FabI.

48. Use as in # 28 or # 29 wherein the composition may be used to treat chloroquine-resistant or any other drug-resistant strain of *Plasmodium falciparum.*

49. A method of inhibiting the growth of malarial parasite by the use of NAV-048, NAV-029, NAV-082, NAV-083, NAV-038, NAV-101, NAV-102, NAV 103, NAV105 or NAV-117 or similar class of inhibitors or any other inhibitor of the enoyl ACP reductase enzyme wherein the method comprises the steps of:

- Monitoring the incorporation of [3H] hypoxanthine in nucleic acid as a quantitative indicator of the inhibition of the parasite growth
- Examining smears of in vitro treated cultures for morphological features of the parasite as an indicator of growth.

50. A method to determine the antimalarial activity of a compound to inhibit the elongation of fatty acid synthesis in a malarial parasite, specifically the enoyl ACP reductase enzyme wherein the method comprises the spectrophotometric measurement of its activity using crotonoyl-CoA, crotonoyl-ACP, enoyl ACP reductase or other intermediates of fatty acid synthesis as substrates. The method comprises the detection of the product of the enzymatic reaction following the separation of the substrate and product by reverse phase-HPLC.

51. A method for screening of drugs using the activity of enoyl ACP reductase as a target for treating a malarial infection comprising the use of a molecular model of enoyl ACP reductase of a malarial parasite.

52. Compounds NAV-048, NAV-029, NAV-082, NAV-083, NAV-038 NAV-101, NAV-102, NAV103, NAV105 or NAV-117 or any other inhibitor of enoyl ACP reductase used in combination with a biocide for treating a malarial or any other parasitic or pathogenic condition.

53. Compounds NAV-048, NAV-029, NAV-082, NAV-083, NAV-038, NAV-101, NAV-102, NAV103, NAV105 or NAV-117 or any compound of the same classes in any pharmaceutical application.

54. Compounds NAV-048, NAV-029, NAV-082, NAV-083, NAV-038, NAV-101, NAV-102, NAV103, NAV105 or NAV-117 or any compound of the same classes in any industrial application.

55. Compounds NAV-048, NAV-029, NAV-082, NAV-083, NAV-038, NAV-101, NAV-102, NAV103, NAV105 or NAV-117 series of compounds in any industrial application.

56. A process of inhibiting Enoyl-CP reductase enzyme to treat malaria and other microbial diseases.

57. A process of inhibiting Enoyl-CP reductase enzyme to treat malaria and other microbial diseases by compounds NAV-048, NAV-029, NAV-082, NAV-083, NAV-038, NAV-101, NAV-102, NAV103, NAV105 or NAV-117 or their analogues.

## REFERENCES

1) WHO. (1999) The World Health Report, 49-63.

2) Sherman, I.W. (Ed.) in Malaria, pp. 1-556. (1998) Am. Soc. Microbiol. Press, Washington, DC.

3) Asindi, A. A., Ekanem, E. E., Ibia, E.O., and Nawangawa, M. A. (1993) Trop. Geogr. Med. 45, 110-113.

4) Surolia, N., and Surolia, A. (2001) Nature Med. 7, 167-173.

5) Kapoor, M., Dar, M. J., Surolia, A., and Surolia N. (2001) Biochem. Biophys. Res. Comm. 289, 832-837.

6) Surolia, N., RamachandraRao, S. R., and Surolia A. (2002) BioEssays, 24, 192-196.

7) Ramya, T. N. C., Surolia, N., and Surolia, A. (2002) Current Science 83, 101-108.

8) Weeks, G., and Wakil, S. J. (1968) J. Biol. Chem. 243, 1180-1189.

9) Marrakchi, H., Zhang, Y.-M., and Rock, C. O. (2002) Biochem. Soc. Trans. 30, 1050-1055.

10) Rock, C. 0., and Cronan, J. E. (1996) Biochim. Biophys. Acta. 1302, 1-16.

11) Turnowsky, F., Fuchs, K., Jeschek, C., and Ho¨genauer, G. (1989) envM genes of Salmonella typhimurium and Escherichia coli. J. Bacteriol. 171, 6555-6565 7.

12) Bergler, H., Wallner, P., Ebeling, A., Leitinger, B., Fuchsbichler, S., Aschauer, H., Kollenz, G., Ho¨genauer, G., and Turnowsky, F. (1994) Protein EnvM is the NADH dependent enoyl ACP reductase (FabI) of Escherichia coli. J. Biol. Chem. 269, 5493-5496.

13) Heath, R. J., and Rock, C. O. (1995) J. Biol. Chem. 44, 26538-26542.

14) Perozzo, R.; Kuo, M,; Sidhu, A.S.; Valiyaveettil, J.T.; Bittman, R.; Jacobs, W.R. Jr.; Fidock, D.A.; Sacchettini, J.C. J. Biol. Chem 2002, 277 (15) 13106-13114.

15) Molecular Operating Environment (MOE 2001.07), Chemical Computing Group Inc.,1255 University St.,Suite 1600,Montreal, Quebec, Canada, H3B 3X3.

16) Goodsell, D. S.; Morris, G. M.; and Olson, A. J. J Mol Recognit. 1996, 9,1-5.

17) Morris, G. M.; Goodsell, D. S.; Halliday, R. S.; Huey, R.; Hart, W. E.; Belew, R. K.; Olson, A. J. J Comput Chem. 1998, 19, 1662.

18) Mehler, E.L. and Solmajer, T. Protein Eng. 1991 Dec;4(8):903-10.

## Claims

**1.** Use of a compound of Formula 7 or a pharmaceutically acceptable salt thereof for preparing a medicament for treating or prophylactically treating an infectious disease.

**2.** Use of a compound of Formula 12 or a pharmaceutically acceptable salt thereof for preparing a medicament for treating or prophylactically treating an infectious disease

**3.** Use according to claim 1 or 2, wherein the infectious disease is malaria.

**4.** Use according to claim 1 or 3, wherein the compound is NAV-048, NAV-029, NAV-101, NAV-102, NAV-103 or NAV-105 or a pharmaceutically acceptable salt thereof.

**5.** Use according to claim 2 or 3, wherein the compound is NAV-083, NAV-082, NAV-038 or NAV-117 or a pharmaceutically acceptable salt thereof.

**6.** A method for identifying an antimalaria compound comprising incubating enoyl ACP reductase with a compound and coenzyme, adding crotonyl-CoA and determing the inhibition of enoyl ACP reductage.

**7.** A composition comprising a compound of Formula 7 or a pharmaceutically acceptable salt thereof and a pharmaceutically acceptable adjuvant, carrier, diluent or excipient.

**8.** A composition comprising a compound of Formula 12 or a pharmaceutically acceptable salt thereof and a pharmaceutically acceptable adjuvant, carrier, diluent or excipient.

**9.** A composition comprising a compound selected from NAV-048, NAV-029, NAV-082, NAV-083, NAV-038, NAV-101, NAV-102, NAV-103, NAV-105 and NAV-117 or a pharmaceutically acceptable salt thereof and a pharmaceutically adjuvant, carrier, diluent or excipient.

**10.** The composition according to anyone of claims 7, 8 or 9, further comprising an antimicrobial or antimalarial compound.

**11.** A method of inhibiting the growth of malarial parasite by the use of NAV-048, NAV-029, NAV-082, NAV-083, NAV-038, NAV-101, NAV-102, NAV-103, NAV-105 and NAV-117 or similar class of inhibitors or any other inhibitor of the enoyl ACP reductase enzyme wherein the method comprises the steps of:

a) monitoring the incorporation of [$^3$H] hypoxanthine in nucleic acid as a quantitative indicator of the inhibition of the parasite growth and
b) examining smears of in vitro treated cultures for morphological features of the parasite as an indicator of growth.

**12.** A method to determine the antimalarial activity of a compound to inhibit the elongation of fatty acid synthesis in a malarial parasite, specifically the enoyl ACP reductase enzyme wherein the method comprises the spectrophotometric measurement of its activity using crotonoyl-CoA, crotonoyl-ACP, enoyl ACP reductase or other intermediates of fatty acid synthesis as substrates.

**13.** A method as in claim 12, wherein the method comprises the detection of the product of the enzymatic reaction following the separation of the substrate and product by reverse phase-HPLC.

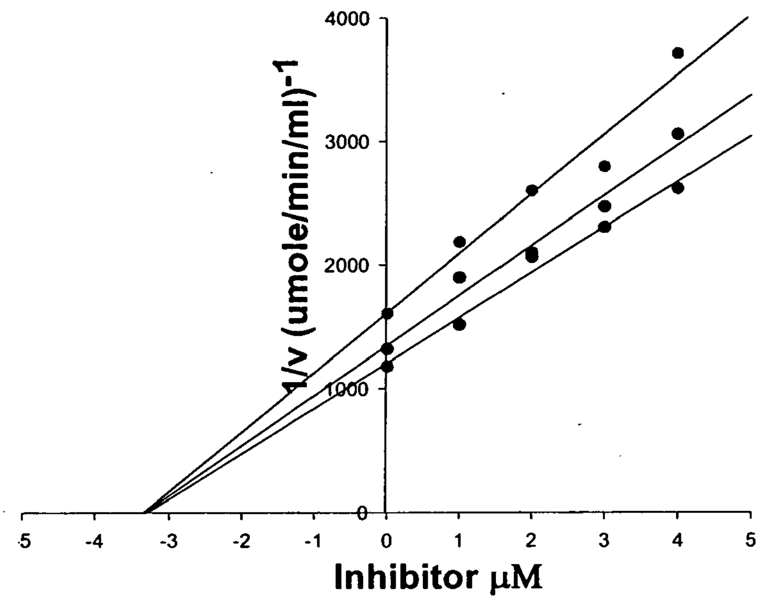
4000
3000
2000
1000
0
1/v (umole/min/ml)-1
-5
-4
-3
-2
-1
0
1
2
3
4
5
Inhibitor μM

Fig.1A(i)

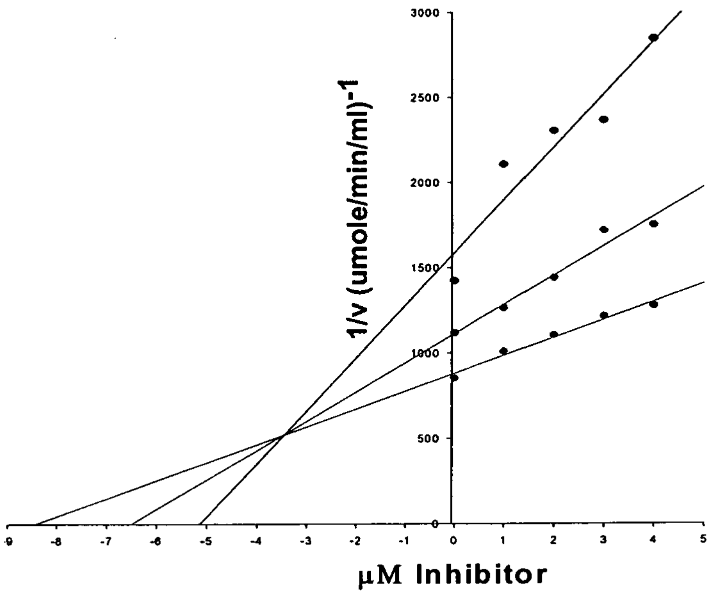
3000
2500
2000
1500
1000
500
0
1/v (umole/min/ml)-1
-9
-8
-7
-6
-5
-4
-3
-2
-1
0
1
2
3
4
5
μM Inhibitor

Fig.1A(ii)

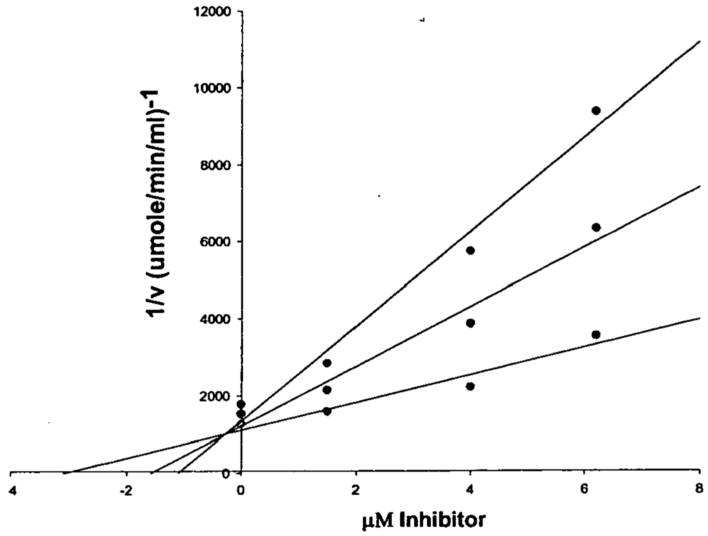
12000
10000
8000
6000
4000
2000
0
1/v (umole/min/ml)-1
4
-2
0
2
4
6
8
μM Inhibitor

Fig.1B(i)

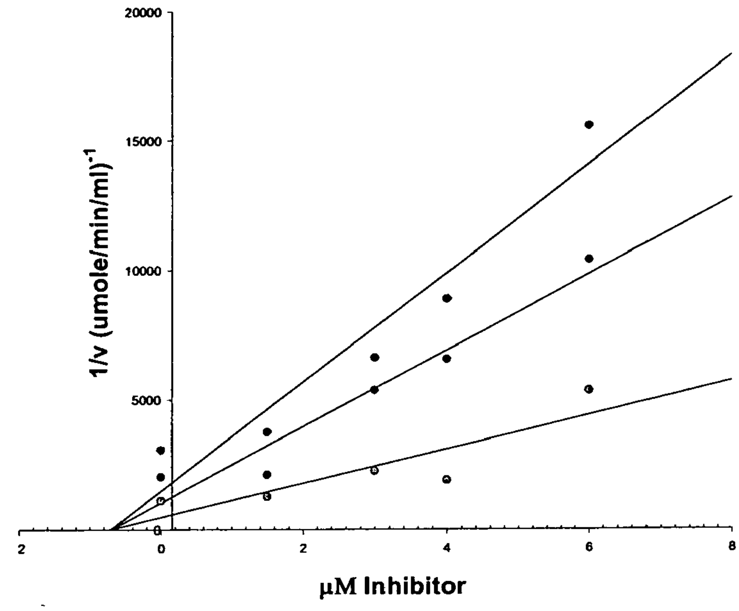
20000
15000
10000
5000
0
1/v (umole/min/ml)-1
2
0
2
4
6
8
μM Inhibitor

Fig.1B(ii)

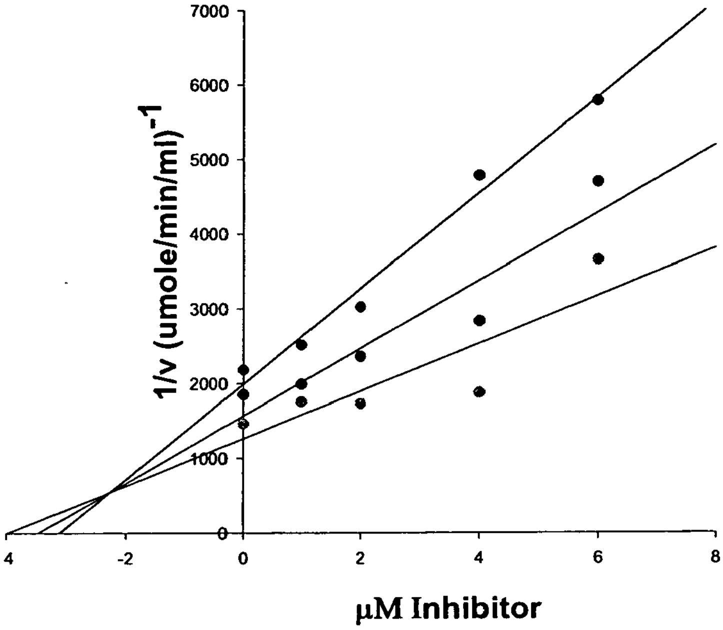
7000
6000
5000
4000
3000
2000
1000
0
1/v (umole/min/ml)-1
4
-2
0
2
4
6
8
μM Inhibitor

Fig.1C(i)

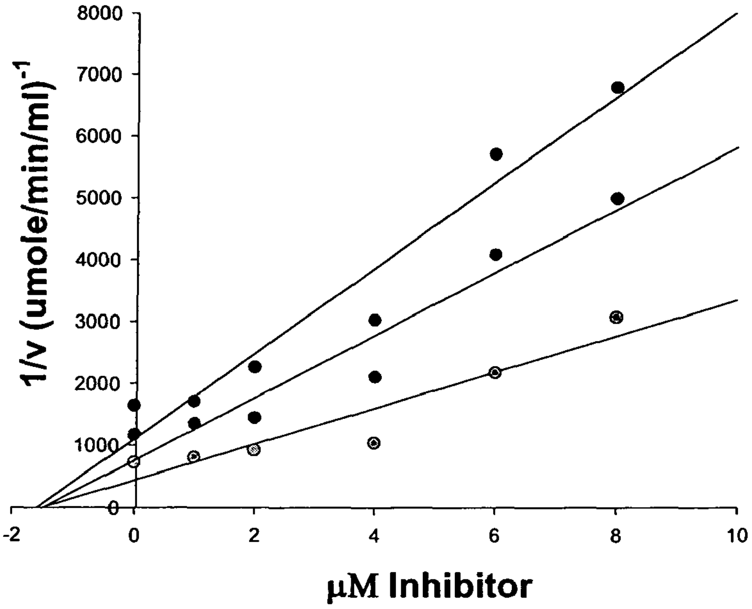
8000
7000
6000
5000
4000
3000
2000
1000
0
1/v (umole/min/ml)-1
-2
0
2
4
6
8
10
µM Inhibitor

Fig.1C(ii)

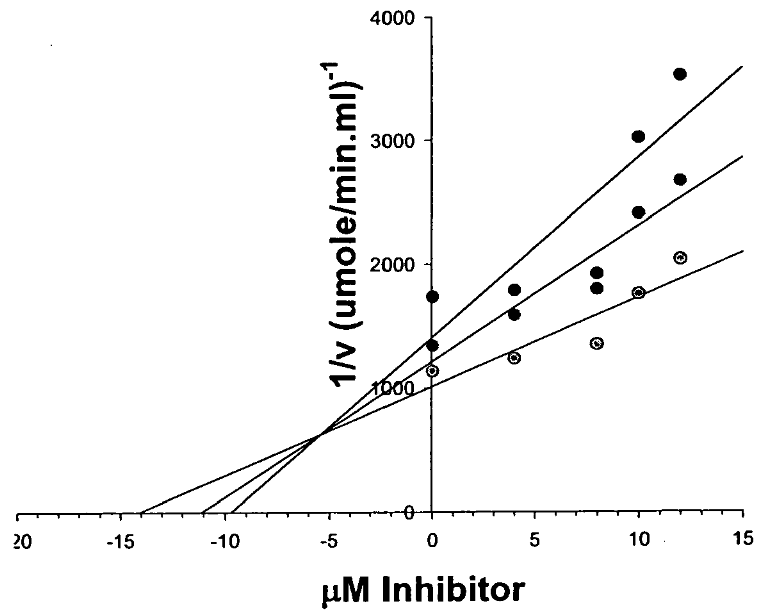
4000
3000
2000
1000
0
1/v (umole/min.ml)-1
20
-15
-10
-5
0
5
10
15
µM Inhibitor

Fig.1D(i)

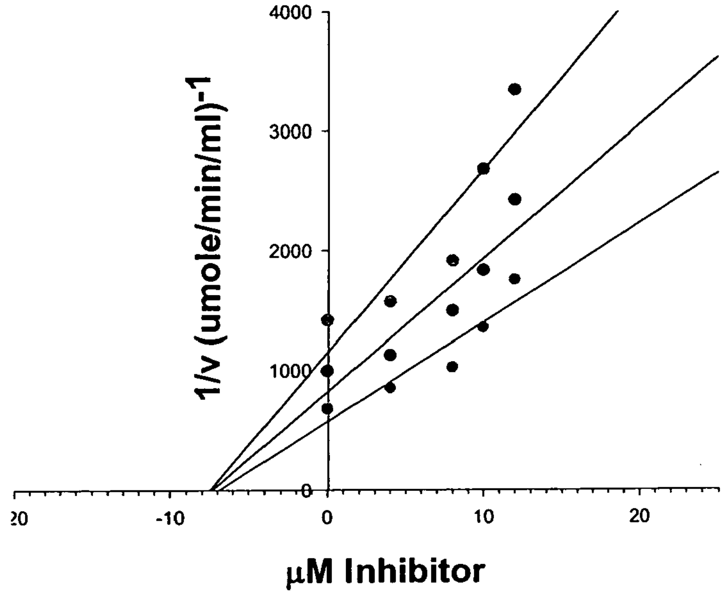
1/v (umole/min/ml)-1
4000
3000
2000
1000
0
20
-10
0
10
20
μM Inhibitor

Fig.1D(ii)

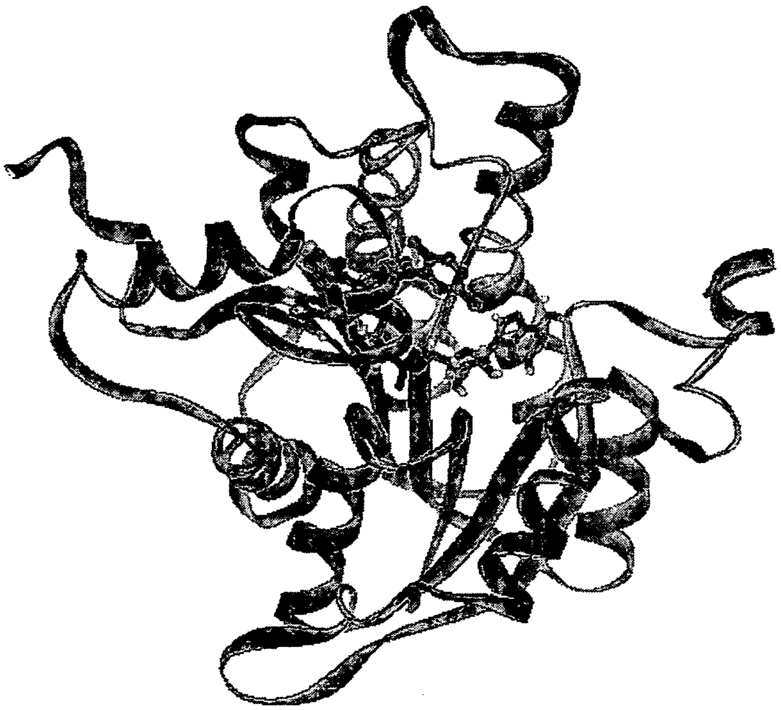

**Fig. 2 (A)** NAV-048 docked with *P. falciparum* Enoyl-ACP reductase.

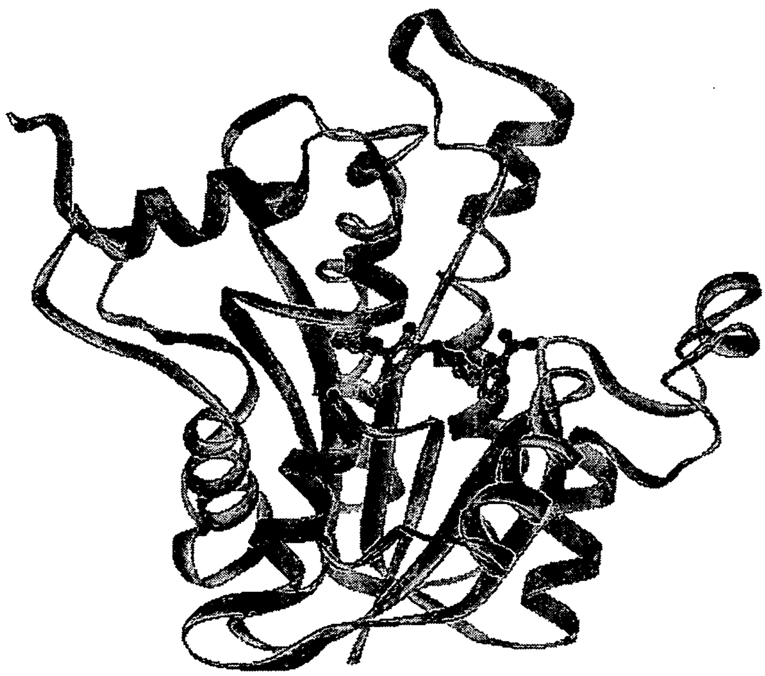

**Fig. 2 (B).** NAV-029 docked with *P. falciparum* Enoyl-ACP reductase.

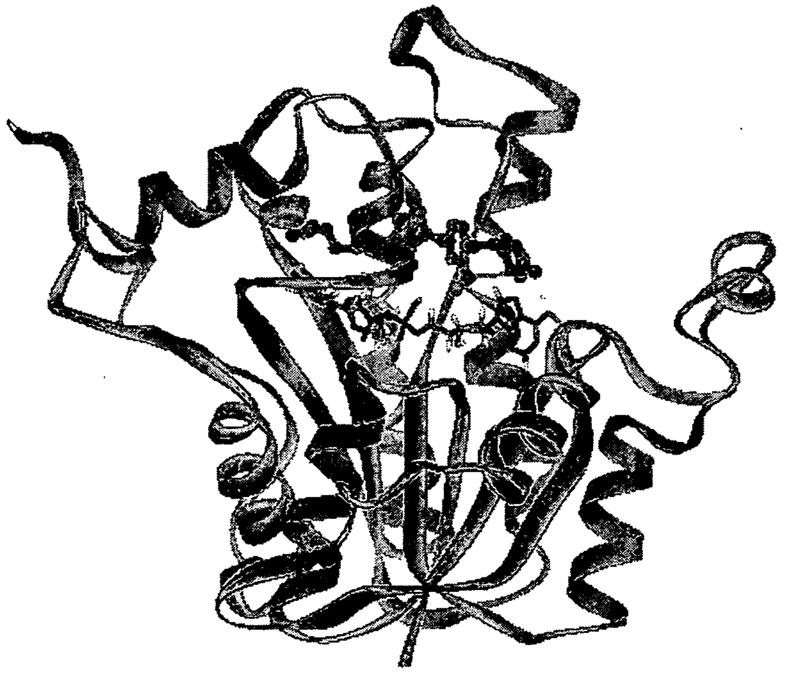

**Fig. 2 (C).** NAV-082 docked with *P. falciparum* Enoyl-ACP reductase.

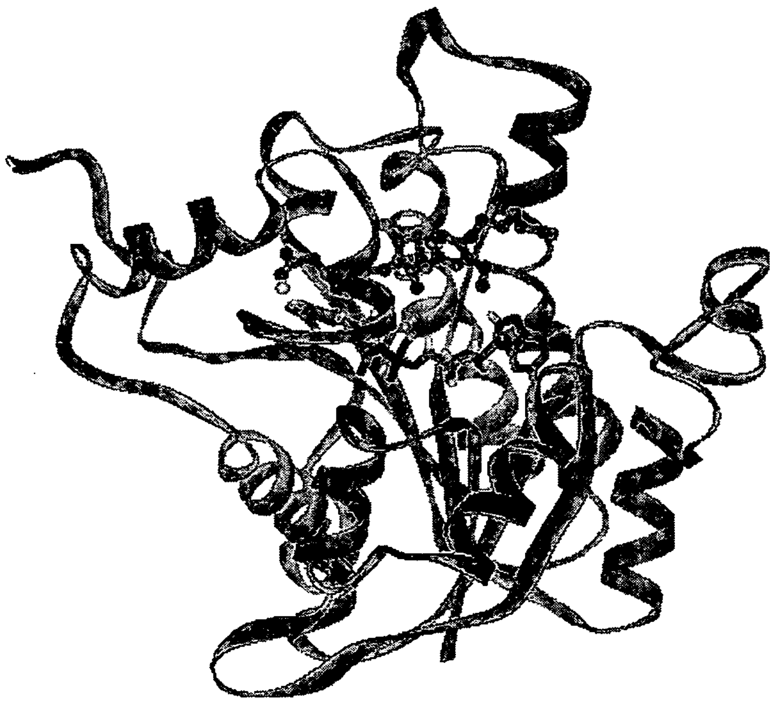

**Fig. 2 (D).** NAV-083 docked with *P. falciparum* Enoyl-ACP reductase.

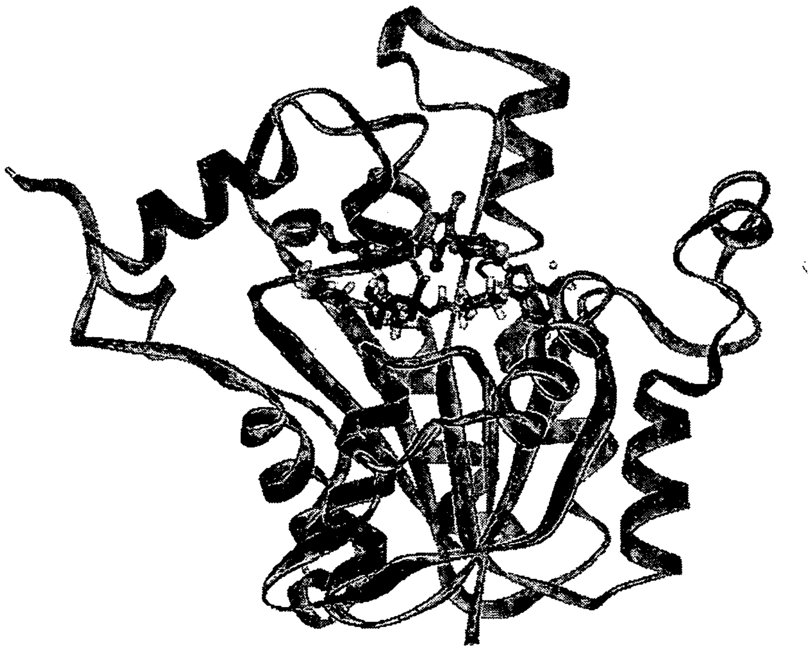

**Fig. 2 (E).** NAV-038 docked with *P. falciparum* Enoyl-ACP reductase.

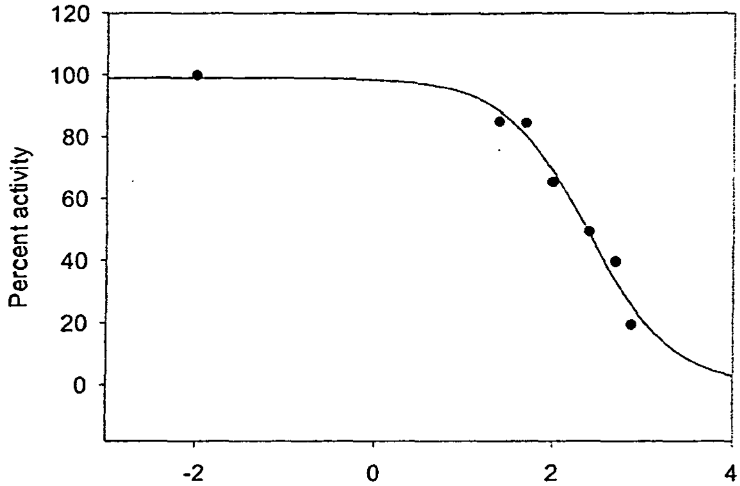


log [NAV-117]nM

Figure 3

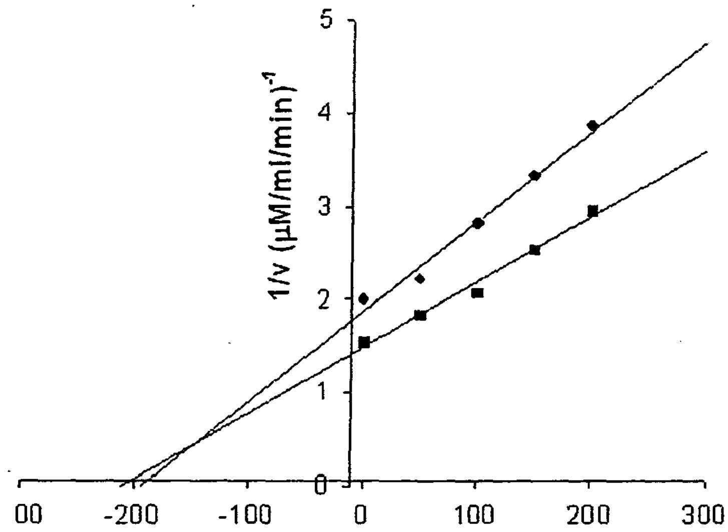


[Compound NAV-117]nM

Figure 4

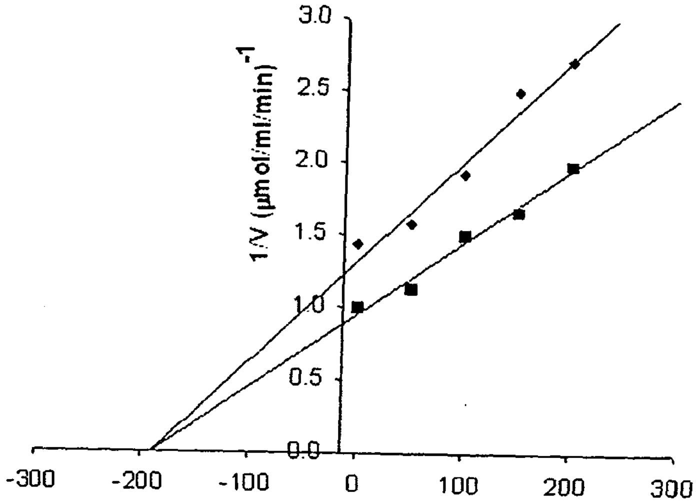


Compound NAV-117 (nM)

Figure 5

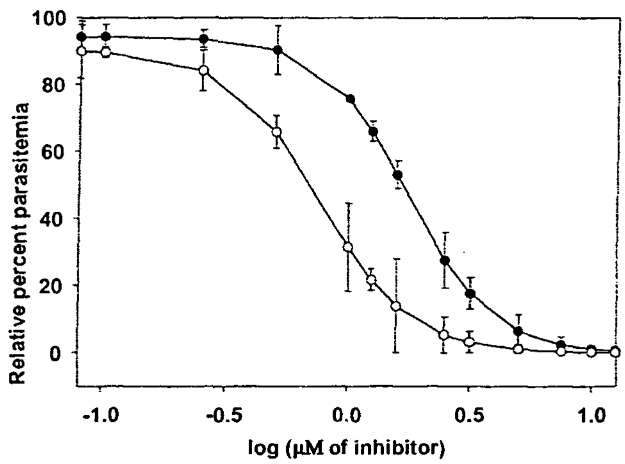
Relative percent parasitemia
100
80
60
40
20
0
-1.0
-0.5
0.0
0.5
1.0
log (μM of inhibitor)

Figure 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **BERTOLINI, G. et al.** *J. Med. Chem.,* 1997, vol. 40, 2011 2016
- **SHAN, D. et al.** *J. Pharm. Sci.,* vol. 86 (7), 765 767
- **BAGSHAWE K.** *Drug Dev. Res.,* 1995, vol. 34, 220 230
- **BODOR, N.** *Advances in Drug Res.,* 1984, vol. 13, 224 331
- **BUNDGAARD, H.** Design of Prodrugs. Elsevier, 1985
- **LARSEN, I. K. et al.** Design and Application of Prodrugs, Drug Design and Development. Harwood Academic Publishers, 1991
- **WHO.** *The World Health Report,* 1999, 49-63
- Malaria. Am. Soc. Microbiol. Press, 1998, 1-556
- **ASINDI, A. A. ; EKANEM, E. E. ; IBIA, E.O. ; NAWANGAWA, M. A.** *Trop. Geogr. Med.,* 1993, vol. 45, 110-113
- **SUROLIA, N. ; SUROLIA, A.** *Nature Med.,* 2001, vol. 7, 167-173
- **KAPOOR, M. ; DAR, M. J. ; SUROLIA, A. ; SUROLIA N.** *Biochem. Biophys. Res. Comm.,* 2001, vol. 289, 832-837
- **SUROLIA, N. ; RAMACHANDRARAO, S. R. ; SUROLIA A.** *BioEssays,* 2002, vol. 24, 192-196
- **RAMYA, T. N. C. ; SUROLIA, N. ; SUROLIA, A.** *Current Science,* 2002, vol. 83, 101-108
- **WEEKS, G. ; WAKIL, S. J.** *J. Biol. Chem.,* 1968, vol. 243, 1180-1189
- **MARRAKCHI, H. ; ZHANG, Y.-M. ; ROCK, C. O.** *Biochem. Soc. Trans.,* 2002, vol. 30, 1050-1055
- **ROCK, C. 0. ; CRONAN, J. E.** *Biochim. Biophys. Acta,* 1996, vol. 1302, 1-16
- **TURNOWSKY, F. ; FUCHS, K. ; JESCHEK, C. ; HO¨GENAUER, G.** envM genes of Salmonella typhimurium and Escherichia coli. *J. Bacteriol.,* 1989, vol. 171, 6555-6565
- **BERGLER, H. ; WALLNER, P. ; EBELING, A. ; LEITINGER, B. ; FUCHSBICHLER, S. ; ASCHAUER, H. ; KOLLENZ, G. ; HO¨GENAUER, G. ; TURNOWSKY, F.** Protein EnvM is the NADH dependent enoyl ACP reductase (FabI) of Escherichia coli. *J. Biol. Chem.,* 1994, vol. 269, 5493-5496
- **HEATH, R. J. ; ROCK, C. O.** *J. Biol. Chem.,* 1995, vol. 44, 26538-26542
- **PEROZZO, R. ; KUO, M ; SIDHU, A.S. ; VALIYAVEETTIL, J.T. ; BITTMAN, R. ; JACOBS, W.R. JR. ; FIDOCK, D.A. ; SACCHETTINI, J.C.** *J. Biol. Chem,* 2002, vol. 277 (15), 13106-13114
- **GOODSELL, D. S. ; MORRIS, G. M. ; OLSON, A. J.** *J Mol Recognit.,* 1996, vol. 9, 1-5
- **MORRIS, G. M. ; GOODSELL, D. S. ; HALLIDAY, R. S. ; HUEY, R. ; HART, W. E. ; BELEW, R. K. ; OLSON, A. J.** *J Comput Chem.,* 1998, vol. 19, 1662
- **MEHLER, E.L. ; SOLMAJER, T.** *Protein Eng.,* December 1991, vol. 4 (8), 903-10